# EUROPEAN PATENT SPECIFICATION

(11) **EP 2 672 973 B1**
(45) Date of publication and mention of the grant of the patent: **30.12.2020**
(21) Application number: 11785058.6
(22) Date of filing: 21.10.2011
(51) Int. Cl.: A61K 31/4439, A61P 25/00, A61P 25/16

(54) **ADMINISTRATION REGIME FOR NITROCATECHOLS**
VERABREICHUNGSPLAN FÜR NITROCATECHOLE
RÉGIME D'ADMINISTRATION DE NITROCATÉCHOLS

(30) Priority: 11.02.2011 US 201161441988 P
(43) Date of publication of application: 18.12.2013
(73) Proprietor: Bial-Portela & CA, S.A., 4745-457 S. Mamede do Coronado (PT)
(72) Inventor: SOARES DA SILVA, Patricio Manuel Vieira Araujo, P-4745-457 S. Mamede do Coronado (PT); NUNES, Teresa Lucia Silva Pereira, P-4745-457 S. Mamede do Coronado (PT); WRIGHT, Lyndon Christopher, P-4745-457 S. Mamede do Coronado (PT); PALMA, Pedro Nuno Leal, P-4745-457 S. Mamede do Coronado (PT); LEARMONTH, David Alexander, P-4745-457 S. Mamede do Coronado (PT)
(74) Representative: Elkington and Fife LLP
(86) International application number: PCT/GB2011/052056
(87) International publication number: WO 2012/107708

(56) References cited:
- WO-A1-2008/094053
- WO-A1-2010/014025
- US-A1- 2010 256 193
- US-A1- 2010 256 194
- FERREIRA JOAQUIM J ET AL: "Opicapone as an adjunct to levodopa in patients with Parkinson's disease and end-of-dose motor fluctuations: a randomised, double-blind, controlled trial.", THE LANCET. NEUROLOGY FEB 2016, vol. 15, no. 2, February 2016 (2016-02), pages 154-165, ISSN: 1474-4465
- FERREIRA J J ET AL: "Effect of opicapone multiple-dose regimens on levodopa pharmacokinetics, motor response, and erythrocyte-COMT activity in Parkinson's patients co-administered with levodopa/dopa-decarboxylase inhibitor", JOURNAL OF NEUROLOGICAL SCIENCES, vol. 333, 22 December 2015 (2015-12-22), XP028721618, ISSN: 0022-510X, DOI: 10.1016/J.JNS.2013.07.390

## Description

This invention relates to the compound 5-[3-(2,5-dichloro-4,6-dimethyl-1-oxy-pyridin-3-yl)-[1,2,4]oxadiazol-5-yl]-3-nitrobenzene-1,2-diol or its pharmacologically acceptable salts, esters, carbamates and phosphates, for use in combination with the catecholamine drug levodopa, for the prophylaxis or treatment of Parkinson's disease, wherein the compound is administered orally in a single daily dose at least one hour before or after the last daily dose of the catecholamine drug.

The rationale for the use of COMT inhibitors as adjuncts to levodopa/aromatic L-amino acid decarboxylase inhibitor (AADCi) therapy is based on their ability to reduce metabolic *O*-methylation of levodopa to 3-*O*-methyl-levodopa (3-OMD). The duration of levodopa-induced clinical improvement is brief as a result of the short *in vivo* half-life of levodopa which contrasts with the long half-life of 3-OMD. Additionally, 3-OMD competes with levodopa for transport across the blood-brain barrier (BBB), which means that only a very limited amount of an orally administered dose of levodopa actually reaches the site of action, i.e. the brain. Commonly, within only a few years of starting levodopa therapy with the usual administration regime, levodopa-induced clinical improvement declines at the end of each dose cycle, giving rise to the so-called 'wearing-off pattern of motor fluctuations. A close relationship between the 'wearing-off' phenomenon and accumulation of 3-OMD has been described (Tohgi, H., et al., Neurosci. Letters, 132:19-22, 1992). It has been speculated that this may result from impaired brain penetration of levodopa due to competition for the transport system across the BBB with 3-OMD (Reches, A. et al., Neurology, 32:887-888, 1982) or more simply that there is less levodopa available to reach the brain (Nutt, J.G., Fellman, J.H., Clin. Neuropharmacol., 7:35-49, 1984). In effect, COMT inhibition protects levodopa from *O*-methylation metabolic breakdown in the periphery, and particularly in the intestine, such that with repeated doses of levodopa, the mean plasma levodopa concentration is raised. In addition to reduced competition for transport into the brain, a significantly greater percentage of the orally administered dose of levodopa is able to reach the site of action. Thus COMT inhibition serves to increase the bioavailability of levodopa and the duration of antiparkinsonian action is prolonged with single adminstrations of levodopa (Nutt, J.G., Lancet, 351:1221-1222, 1998).

The most potent COMT inhibitors reported thusfar are 3,4-dihydroxy-4'-methyl-5-nitrobenzophenone (Tolcapone, Australian pat. AU-B-69764/87) and (E)-2-cyano-N,N-diethyl-3-(3,4-dihydroxy-5-nitrophenyl)acrylamide (Entacapone, German pat. DE 3740383 A1).

Although sharing essentially the same pharmacophore, tolcapone differs from entacapone in that it easily enters the central nervous systems (CNS) and is able to inhibit cerebral COMT as well as peripheral COMT. Shortly after its launch, tolcapone was withdrawn from the market after several cases of hepatotoxicity were reported including three unfortunate deaths from fatal fulminant hepatitis. Today tolcapone can only be used in Parkinsonian patients who are unresponsive to other treatments and only with regular monitoring of liver function, which is expensive and inconvenient for the patient. Although the actual mechanistic causes of the liver toxicity associated with tolcapone are not fully understood, *in vitro* studies have shown that tolcapone may be reduced metabolically to reactive intermediates and it has been speculated that these may form covalent adducts with hepatic proteins resulting in hepatocellular injury (Smith, K.S. et al, Chem. Res. Toxicol., 16:123-128, 2003).

Entacapone on the other hand, although sharing the same nitrocatechol pharmacophore with tolcapone, is not associated with liver toxicity and is generally regarded as a safe drug. Unfortunately, however, entacapone is a significantly less potent COMT inhibitor than tolcapone and has a much shorter *in-vivo* half-life. This means that entacapone has a very limited duration of effect and as a consequence, the drug must be administered in very high doses with every dose of levodopa taken by the patient. As such, the clinical efficacy of entacapone has been questioned - indeed a recent study (Parashos, S.A. et al., Clin. Neuropharmacol., 27(3): 119-123, 2004) revealed that the principal reason for discontinuation of entacapone treatment in Parkinson's disease patients was a perceived lack of efficacy.

Furthermore, the relatively short *in-vivo* half-life of known COMT inhibitors requires continuous treatment regimens normally involving the administration of several doses a day which many patients find to be burdensome. For example, tolcapone has to be administered three times a day. This factor can therefore interfere with patient compliance and quality of life.

Accordingly, there is still a need for COMT inhibitors exhibiting balanced properties of bioactivity, bioavailability and safety. In particular, there is a need for COMT inhibitors having a long *in-vivo* half-life and, thus, a prolonged action on COMT enabling fewer dosages to obtain the desired therapeutic effect.

The applicant has previously discovered compounds which, despite having a relatively short half life, are very potent COMT inhibitors endowed with exceptionally long duration of action compared to COMT inhibitors in the prior art (see WO2007/013830).

These compounds, which are shown hereinbelow as compounds of general formula (I), also markedly enhance the bioavailability of levodopa and increase the delivery of levodopa to the brain. The compounds significantly augment the levels of dopamine in the brain over a long period of time.

The compounds of formula (I) are 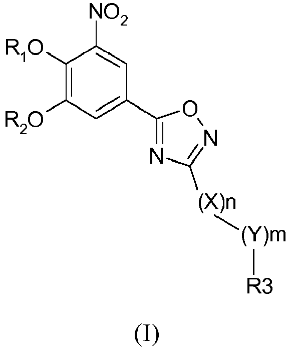 where R₁ and R₂ are the same or different and signify hydrogens, groups hydrolysable under physiological conditions, or optionally substituted alkanoyls or aroyls; X signifies a methylene group; Y represents O, S or NH; n represents 0, 1, 2 or 3; m represents 0 or 1; R₃ signifies a pyridine N-oxide group according to the formula A, B, or C, which is connected as indicated by the unmarked bond: 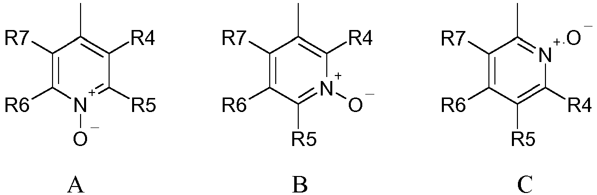 where R₄, R₅, R₆ and R₇ are the same or different, and signify hydrogen, alkyl, thioalkyl, alkoxy, aryloxy, thioaryl, alkanoyl, aroyl, aryl, amino, alkylamino, dialkylamino, cycloalkylamino, heterocycloalkylamino, alkylsulphonyl, arylsulphonyl, halogen, haloalkyl, trifluoromethyl, cyano, nitro or heteroaryl; or two or more of R₄, R₅, R₆ and R₇ taken together signify aliphatic or heteroaliphatic rings or aromatic or heteroaromatic rings; the term 'alkyl', including its variant 'alk-' in terms such as 'alkoxy', 'alkanoyl' mean carbon residues, straight or branched, containing from one to six carbon atoms; the term 'aryl' means a phenyl or naphthyl group; the term 'heterocycloalkyl' represents a four to eight-membered cyclic ring optionally incorporating at least one atom of oxygen, sulphur or nitrogen; the term 'heteroaryl' represents a five or six-membered ring incorporating at least one atom of sulphur, oxygen or nitrogen; the term 'halogen' represents fluorine, chlorine, bromine or iodine; and if R₄, R₅, R₆, and R₇ represent alkyl or aryl they are optionally substituted by one or more hydroxy, alkoxy or halogen groups; or a pharmaceutically acceptable salt, ester, carbamate or phosphate thereof.

Even more surprisingly, the increased levels of levodopa are maintained steady over extended periods of time. These sustained effects upon both COMT activity and levodopa bioavailability after the administration of compounds of general formula (I) are markedly greater than those observed with tolcapone, the only COMT inhibitor thusfar known to be endowed with a reasonably long duration of action. (Tolcapone has a terminal half life of around 2 hours and must be administered around 3 times per day.) Furthermore, compounds of general formula (I) produce a steady increase in levodopa delivery to the brain over extended periods of time, which contrasts with that observed with tolcapone, which is prone to induce marked oscillations in the brain delivery of levodopa. Thus compounds of general formula (I) are more likely to be endowed with therapeutic advantages due to sustained constant elevation of levodopa levels whilst the use of tolcapone is likely to induce undesirable side-effects such as dyskinesia due to abrupt increases and decreases in levodopa levels.

The present invention is based on a surprisingly advantageous administration regimen for the administration of a compound of formula (I) which maximises the COMT inhibitory effect of the compounds.

Accordingly, in a first aspect the present invention relates to the compound 5-[3-(2,5-dichloro-4,6-dimethyl-1-oxy-pyridin-3-yl)-[1,2,4]oxadiazol-5-yl]-3-nitrobenzene-1,2-diol or its pharmacologically acceptable salts, esters, carbamates and phosphates, for use in combination with the catecholamine drug levodopa, for the prophylaxis or treatment of Parkinson's disease, wherein the compound is administered orally in a single daily dose at least one hour before or after the last daily dose of the catecholamine drug.

In a second aspect the present invention relates to the compound 5-[3-(2,5-dichloro-4,6-dimethyl-1-oxy-pyridin-3-yl)-[1,2,4]oxadiazol-5-yl]-3-nitrobenzene-1,2-diol or its pharmacologically acceptable salts, esters, carbamates and phosphates, for use in combination with the catecholamine drug levodopa, for the prophylaxis or treatment of Parkinson's disease, wherein the 5-[3-(2,5-dichloro-4,6-dimethyl-1-oxy-pyridin-3-yl)-[1,2,4]oxadiazol-5-yl]-3-nitrobenzene-1,2-diol or its pharmacologically acceptable salt, ester, carbamate or phosphate compound is administered orally once daily at least one hour before or after the last daily dose of the catecholamine drug, and less than 90 minutes before going to bed or at bedtime, and at least one hour after the most recent intake of food and at least one hour before the next intake of food.

Surprisingly, the applicant has found that the compound is advantageously administered to a patient who has a digestive system which is as free from food as possible. Therefore, the compound should preferably be administered to a patient without food and/or between intakes of food e.g. between meals, prior to sleep, before bedtime or at bedtime. Administering the compound in this manner results in the compound having better activity e.g. more long lasting and increased inhibition of COMT.

Further, as mentioned, the COMT inhibitors of formula (I) are used as an adjunct to catecholamine therapy, so that the metabolism of the catecholamine drug by COMT is decreased. The applicant has also surprisingly found that when the compound is administered to a patient already taking the catecholamine levodopa (L-DOPA), the effects of the compound are improved if the compound is administered sequentially with the catecholamine. In particular, it has surprisingly been discovered that the compound adversely affects the bioavailability of levodopa, and levodopa adversely affects the bioavailability of the compound. Consequently, according to the invention the compound is administered at least one hour before or after the last daily dose of levodopa has been given to the patient and before the following day's dosage of levodopa is administered. Therefore, the compound and the catecholamine drug are not within the patient's digestive system at the same time and/or not being substantially absorbed at the same time.

For the purpose of the present invention, last daily dose, last dose of the day, last daily administration and last administration of the day have the same meaning and can be used interchangeably.

The present invention will now be described with reference to the accompanying drawings.
Figure 1 shows mean plasma concentration-time profile of 5-[3-(2,5-dichloro-4,6-dimethyl-1-oxy-pyridin-3-yl)-[1,2,4]oxadiazol-5-yl]-3-nitrobenzene-1 ,2-diol (COMPOUND A) following fasting and fed conditions.
Figure 2 shows mean levodopa plasma concentration-time profiles following single oral administration of Sinemet® (levodopa/carbidopa) 100/25 mg administered alone, administered with 50 mg COMPOUND A separated 1 h and administered concomitantly with 50 mg COMPOUND A.
Figure 3 shows mean S-COMT activity (metanephrine formed, pmol/mg protein/h) profiles from baseline (pre-dose) following single oral administration of Sinemet® 100/25 administered alone, administered with 50 mg COMPOUND A separated 1 h, administered concomitantly with 50 mg COMPOUND A and when COMPOUND A was administered alone.

As mentioned, because the compound is extremely potent, it can be administered in a single daily administration.

The compound is preferably administered less than 90 minutes before going to bed or at bedtime. The compound is preferably administered less than 60 minutes before going to bed or less than 30 minutes before going to bed. The term at bedtime means less than 5 minutes before bedtime, for example on going to bed.

In other words, the compound is taken by the patient before the patient goes to bed (i.e. before bedtime or at bedtime), e.g. less than 90 minutes before bedtime, particularly less than 60 minutes before bedtime, less than 30 minutes before bedtime or less than 5 minutes before bedtime.

As will be clear in the context of the invention, the term 'before bedtime' does not mean any time in the day prior to going to bed, and in particular does not include, for example, 12 hours before going to bed. Rather this term means the drug is taken in the period close to the patient going to sleep and probably as part of the patient's bedtime routine.

In one embodiment, the compound is administered in combination therapy with the catecholamine drug levodopa.

As such, the administration regimen of the compound and the catecholamine drug differ: each will be administered at different times. It will therefore be appreciated that the compounds of the combination will be administered sequentially (e.g. before or after), in different pharmaceutical formulations (i.e. separately). The administration regime of each of the two or more compounds in a combination therapy may also differ with respect to the route of administration.

The applicant has surprisingly discovered that the compound and the catecholamine drug each adversely affect the bioavailability of the other.

In particular, the compound is administered at least one hour before or after the last administration of the day of the catecholamine drug and before the first administration of the next day of the catecholamine drug. This therefore avoids the adverse consequence each drug has on the bioavailability of the other. Preferably the COMT inhibitory activity of the compound is active prior to administration of the catecholamine drug.

In one embodiment, the compound is administered from 60 to 150 minutes before or after the last daily administration of the catecholamine drug. For example the compound of formula (I) is administered at least one hour, before or after the last daily administration of the catecholamine drug.

In another embodiment, the compound is administered once daily at least one hour before or after the last daily administration of the catecholamine drug. In embodiments of the invention there is a period of at least two, three, four, five or six hours between the administration of the catecholamine and the compound.

Preferably the subsequent administration of the catecholamine is administered at least two, more preferably at least three, and most preferably at least six hours after administration of the compound. Suitably, the subsequent administration of the catecholamine is administered 12 hours or 23 hours after administration of the compound. Preferably, the subsequent administration of the catecholamine drug is the first daily dose of catecholamine drug of the next day.

In addition, the administration regime according to the invention involves administration of the compound when the patient has a digestive system free from food. The applicant has discovered that the compounds have improved bioavailability when administered to a patient when the patient does not have food in their digestive system. In particular, the compound should be administered to the patient less than 90 minutes before going to bed or at bedtime, without food after the patient has had their final meal of the day.

The term "a digestive system free from food" means that the part of the digestive system where most of the absorption of the compound occurs is free from food, e.g. stomach, small intestine (duodenum, jejunum, ileum).

In one embodiment of the invention the compound is administered at least one hour after the most recent intake of food and at least one hour before the next intake of food.

In one embodiment of the invention the compound is administered from 0.25 to 12 hours, preferably from 0.5 to 6 hours, more preferably from 0.75 to 4 hours, after an intake of food. In one embodiment of the invention the compound is administered after 0.25 to 10 hours overnight fasting.

In one embodiment of the invention, the compound is administered from 0.25 to 2 hours, preferably from 0.5 to 1.5 hours, before an intake of food.

Most preferably, in order to avoid the interaction between the compound and the catecholamine drug, and also to administer the compound when the patient has a digestive system free of food, the compound is administered once daily less than 90 minutes before going to bed or at bedtime.

As used herein, the term 'effective daily dose' is the effective daily amount of compound administered when administered according to the dosing periodicity.

In the present invention, effective daily doses of the compound are in the range of about 1 to about 1200 mg/day, preferably about 1 to about 900 mg/day, more preferably about 5 to about 400 mg/day, even more preferably about 25 to about 300 mg/day, for example specific daily doses of 1 mg, 3 mg, 5 mg, 10 mg, 15 mg, 20 mg, 25 mg, 30 mg, 50 mg, 100 mg, 200 mg, 400 mg, 800 mg or 1200 mg.

As used herein, the term "dosage unit" refers to the amount of compound administered in each dosing periodicity.

It is preferred that individual dosage units of the compound are in the range of about 1 to about 2400 mg, more preferably about 1 to about 1200 mg, even more preferably about 1 to about 800 mg, for example 1 mg, 3 mg, 5 mg, 10 mg, 15 mg, 20 mg, 25 mg, 30 mg, 50 mg, 100 mg, 200 mg, 400 mg, 800 mg or 1200 mg.

As mentioned above, COMT inhibitors are often used as adjuncts to catecholamine compounds because they reduce their metabolic *O*-methylation. In particular, COMT inhibitors are often used as adjuncts to levodopa/aromatic L-amino acid decarboxylase inhibitor (AADCi) therapy because they reduce metabolic O-methylation of levodopa to 3-*O*-methyl-levodopa (3-OMD).

Therefore, preferably, the pathological states treated by the compounds are central and peripheral nervous system-associated disorders of humans which benefit from administration of a COMT inhibitor.

When the compound is administered in combination with a catecholamine drug, it is possible that the catecholamine drug is administered sequentially or concomitantly with an AADCi, in particular cardidopa or benserazide.

The compound, the catecholamine drug and the AADCi may be administered separately or in any combination. They may be administered concomitantly (for example, simultaneously) or sequentially, and with the same or differing dosing periodicity.

The use of the compounds according to the invention is for the propylaxis or treatment of Parkinson's Disease.

As used herein, the term treatment and variations such as 'treat' or 'treating' refer to any regime that can benefit a human or non-human animal. In addition the compound can be used for prophylaxis (preventative treatment). Treatment may include curative, alleviation or reducing effects, such effects relating to one or more of the symptoms associated with the central and peripheral nervous system-associated disorders.

One particular embodiment of the invention that can be mentioned is the compound 5-[3-(2,5-dichloro-4,6-dimethyl-1-oxy-pyridin-3-yl)-[1,2,4]oxadiazol-5-yl]-3-nitrobenzene-1,2-diol and its pharmacologically acceptable salts, esters, carbamates and phosphates, for use in combination with the catecholamine drug levodopa, for the prophylaxis or treatment of Parkinson's disease, wherein the compound is administered orally once daily at least one hour before or after the last daily dose of the catecholamine drug and less than 90 minutes before going to bed or at bedtime and/or without food and/or between intakes of food and/or at least one hour after the most recent intake of food and at least one hour before the next intake of food.

Also described herein is a method for inhibiting COMT in a subject, comprising administering, without food and/or between intakes of food and/or prior to sleep and/or before bedtime and/or at bedtime and/or before or after administration of a catecholamine drug, an effective dose of the compound as defined above to the subject.

Also described herein is a method for increasing levels of levodopa in the brain of a subject being treated with levodopa, comprising administering, without food and/or between intakes of food and/or prior to sleep and/or before bedtime and/or at bedtime and/or before or after administration of a catecholamine drug, an effective dose of the compound as defined above to the subject.

Also described herein is a method for increasing levels of levodopa in the plasma of a subject being treated with levodopa, comprising administering, without food and/or between intakes of food and/or prior to sleep and/or before bedtime and/or at bedtime and/or before or after administration of a catecholamine drug, an effective dose of the compound as defined above to the subject.

Also described herein is a method for decreasing levels of 3-*O*-methyl-levodopa (3-OMD) in the brain of a subject being treated with levodopa, comprising administering, without food and/or between intakes of food and/or prior to sleep and/or before bedtime and/or at bedtime and/or before or after administration of a catecholamine drug, an effective dose of the compound as defined above to the subject.

Also described herein is a method for decreasing levels of 3-OMD in the plasma of a subject being treated with levodopa, comprising administering, without food and/or between intakes of food and/or prior to sleep and/or before bedtime and/or at bedtime and/or before or after administration of a catecholamine drug, an effective dose of the compound as defined above to the subject.

Also described herein is a method for increasing bioavailability of levodopa in the brain of a subject being treated with levodopa, comprising administering, without food and/or between intakes of food and/or prior to sleep and/or before bedtime and/or at bedtime and/or before or after administration of a catecholamine drug, an effective dose of the compound as defined above to the subject.

Also described herein is method for increasing bioavailability of levodopa in the plasma of a subject being treated with levodopa, comprising administering, without food and/or between intakes of food and/or prior to sleep and/or before bedtime and/or at bedtime and/or before or after administration of a catecholamine drug, an effective dose of the compound as defined above to the subject.

Also described herein is a package comprising a pharmaceutical composition of the compound in combination with instructions to administer said formulation without food and/or between intakes of food and/or prior to sleep and/or before bedtime and/or at bedtime and/or before or after administration of a catecholamine drug.

The compound 5-[3-(2,5-dichloro-4,6-dimethyl-1-oxy-pyridin-3-yl)-[1,2,4]oxadiazol-5-yl]-3-nitrobenzene-1,2-diol is also designated herein as compound A, and its pharmacologically acceptable salts, esters, carbamates and phosphates. The half life of compound A is relatively short given its long duration of action.

Details of the preparation of compounds of general formula (I) can be found in WO2007/013830A1.

The compound may also be present in the form of pharmacologically acceptable salts, esters, carbamates or phosphates thereof. Suitable pharmaceutically acceptable counter ions are known to the art.

It is also possible to use prodrugs of the compound in order to alter the therapeutic profile of the active compound.

The compound is administered as a pharmaceutical composition. For the preparation of pharmaceutical compositions of the compound, inert pharmaceutically acceptable carriers are admixed with the active compounds. The pharmaceutically acceptable carriers may be solid or liquid. Solid form preparations include powders, tablets, dispersible granules and capsules. A solid carrier can be one or more substances which may also act as diluent, flavouring agent, solubiliser, lubricant, suspending agent, binder, glidant, or disintegrant; it may also be an encapsulating material.

Preferably the pharmaceutical composition is in unit dosage form, e.g. a packaged preparation, the package containing discrete quantities of the preparation, for example packaged tablets, capsules and powders in vials or ampoules.

In general, the compound is administered orally.

Other aspects of the invention are as defined in the claims.

### Examples

### Example 1: Preparation of compound A

### (5-[3-(2,5-Dichloro-4,6-dimethyl-1-oxy-pyridin-3-yl)-[1,2,4]oxadiazol-5-yl]-3-nitrobenzene-1,2-diol)

a) To a stirred solution of 3,4-dibenzyloxy-5-nitrobenzoic acid (0.50 g, 1.319 mmol) in dimethylformamide (5 mL) at room temperature was added 1,1-carbonyldiimidazole (0.24 g, 1.45 mmol) in one portion. After stirring for ninety minutes, 2,5-dichloro-N'-hydroxy-4,6-dimethylnicotinamide (0.40 g, 1.45 mmol) was added in one portion. The resulting mixture was stirred at 135 °C for five hours and then at room temperature overnight. The reaction mixture was poured onto ice-2 N HCl (100 mL) and the resulting precipitate was filtered off, washed with water and dried in air. Recrystallisation from isopropanol gave a pale yellow solid (0.55 g, 72 %).
b) To a stirred solution of the solid obtained above (0.50 g, 0.866 mmol) in dichloromethane (20 mL) was added urea-hydrogen peroxide addition complex (0.41 g, 4.33 mmol) in one portion. The mixture was cooled in an ice-water bath and administration of a catecholamine drug, an effective dose of a compound of general formula (I) as defined above to the subject.

The present invention also relates to a package comprising a pharmaceutical composition of a compound of the general formula (I) in combination with instructions to administer said formulation without food and/or between intakes of food and/or prior to sleep and/or before bedtime and/or at bedtime and/or before or after administration of a catecholamine drug.

For the compounds of formula (I), the following definitions are preferred.

Groups hydrolysable under physiological conditions represent groups cleavable *in vivo,* at physiological conditions of pH and temperature. On page 1354 of the 6th edition of "Foye's Principles of Medicinal Chemistry", 2006, eds. Wolter Kluwer, the pH values for tissue fluids are indicated. Examples of groups hydrolysable under physiological conditions for the -OH functional group are esters, carbamates and phosphates. Further examples of groups hydrolysable under physiological conditions for the -OH functional group are well known to the skilled in the art and can be found for example on pages 101-103 from Korolkovas in Essentials of Medicinal Chemistry, 2nd edn., 1988, eds. John Wiley & Sons and on page 426 of Krogsgaard-larsen et al in Textbook of Drug Design and Discovery, 3rd edn., 2002, eds. Taylor & Francis.

Preferably, R₄, R₅, R₆ and R₇ independently from each other represent hydrogen, C₁-C₆-alkyl, C₆-C₁₂ aryl, C₁-C₆-thioalkyl, C₁-C₆alkoxy, C₆-C₁₀-aryloxy, C₆-C₁₀-thioaryl, C₁-C₆-alkanoyl, C₇-C₁₁-aroyl, amino, C₁-C₆-alkylamino, di-C₁-C₆-alkylamino, C₃-C₁₂-cycloalkylamino, C₄-C₈-heterocycloalkylamino, C₁-C₆-alkylsulphonyl, C₆-C₁₀-arylsulphonyl, halogen, C₁-C₆-haloalkyl, trifluoromethyl, cyano, nitro or heteroaryl.

When R₄, R₅, R₆, and/or R₇ represent C₁-C₆-alkyl residues, preferably R₄, R₅, R₆, and/or R₇ represent methyl, ethyl, n-propyl, isopropyl, n-butyl, sec-butyl, tert-butyl, pentyl, or hexyl.

When R₄, R₅, R₆ and/or R₇ represent C₆-C₁₂-aryl residues, preferably R₄, R₅, R₆ and/or R₇ represent phenyl or naphthyl.

When R₄, R₅, R₆ and/or R₇ represent C₁-C₆-thioalkyl residues, preferably R₄, R₅, R₆ and/or R₇ represent thiomethyl, thioethyl, thio-n-propyl, thio-isopropyl, thio-n-butyl, thio-n-pentyl, or thio-n-hexyl.

When R₄, R₅, R₆ and/or R₇ represent C₁-C₆-alkoxy residues, preferably R₄, R₅, R₆ and/or R₇ represent methoxy, ethoxy, n-propoxy, isopropoxy, n-butoxy, sec-butoxy or tert-butoxy.

When R₄, R₅, R₆ and/or R₇ represent C₆-C₁₀-aryloxy residues, preferably R₄, R₅, R₆ and/or R₇ represent phenoxy or naphthoxy.

When R₄, R₅, R₆ and/or R₇ represent C₆-C₁₀-thioaryl residues, preferably R₄, R₅, R₆ and/or R₇ represent thiophenyl or thionaphthyl.

When R₄, R₅, R₆ and/or R₇ represent C₁-C₆-alkanoyl residues, preferably R₄, R₅, R₆ and/or R₇ represent methanoyl, ethanoyl, propanoyl or butanoyl.

When R₄, R₅, R₆ and/or R₇ represent C₇-C₁₁-aroyl residues, preferably R₄, R₅, R₆ and/or R₇ represent benzoyl or naphthoyl.

When R₄, R₅, R₆ and/or R₇ represent C₁-C₆-alkylamino residues, preferably R₄, R₅, R₆ and/or R₇ represent methylamino, ethylamino, n-propylamino, isopropylamino or n-butylamino.

When R₄, R₅, R₆ and/or R₇ represent di-C₁-C₆-alkylamino residues, preferably R₄, R₅, R₆ and/or R₇ represent dimethylamino, diethylamino, di-n-propylamino, di-n-butylamino, di-isopropylamino, methylethylamino, methylpropylamino or ethylpropylamino.

When R₄, R₅, R₆ and/or R₇ represent C₃-C₁₂-cycloalkylamino residues, preferably R₄, R₅, R₆ and/or R₇ represent pyrrolidino, piperidino, cyclohexylamino or dicyclo hexylamino.

When R₄, R₅, R₆ and/or R₇ represent C₄-C₈-heterocycloalkylamino residues, preferably R₄, R₅, R₆ and/or R₇ represent morpholino, 2,6-dimethylmorpholino, 3,5-dimethylmorpholino, piperazino, N-methylpiperazino or N-ethylpiperazino.

When R₄, R₅, R₆ and/or R₇ represent C₁-C₆-alkylsulphonyl or C₆-C₁₀-arylsulphonyl residues, preferably R₄, R₅, R₆ and/or R₇ represent methylsulfonyl, ethylsulfonyl, phenylsulfonyl, or tolylsulfonyl.

When R₄, R₅, R₆ and/or R₇ represent halogen residues, preferably R₄, R₅, R₆ and/or R₇ represent chloro, bromo, iodo or fluoro.

When R₄, R₅, R₆ and/or R₇ represent C₁-C₆-haloalkyl residues, preferably R₄, R₅, R₆ and/or R₇ represent chloromethyl, fluoromethyl, dichloromethyl, difluoromethyl, trichloromethyl or trifluoromethyl.

When R₄, R₅, R₆ and/or R₇ represent heteroaryl residues, preferably R₄, R₅, R₆, and/or R₇ represent pyridyl, pyrimidyl, isoxazolyl, oxazolyl, isoxadiazolyl, oxadiazolyl, triazolyl or tetrazolyl.

When two or more of residues R₄, R₅, R₆ and R₇ taken together represent aliphatic or heteroaliphatic rings or aromatic or heteroaromatic rings, the two or more residues preferably represent aliphatic or heteroaliphatic rings or aromatic or heteroaromatic rings. Preferred combined residues are indolizinyl, isoindolyl, indolyl, indazolyl, purinyl, quinolizinyl, naphthyridinyl, isoquinolyl and quinolyl.

Where they represent aryl or alkyl, the above substituents R₄, R₅, R₆ and R₇ may optionally be substituted one or more times by hydroxy, alkoxy or halogen groups.

In the present description of medical indications, treatments and dosing regimens for pharmaceutical compositions containing compounds according to general formula (I) of the invention, the most preferred example of a compound according to the general formula (I) is 5-[3-(2,5-dichloro-4,6-dimethyl-1-oxy-pyridin-3-yl)-[1,2,4]oxadiazol-5-yl]-3-nitrobenzene-1,2-diol, henceforth designated as compound A, and its pharmacologically acceptable salts, esters, carbamates and phosphates. The half life of compound A is relatively short given its long duration of action.

Other preferred compounds of the above general formula (I) for use in the subsequent medical indications, treatments and dosing regimens include 3-(3-(3,4-dihydroxy-5-nitrophenyl)-1,2,4-oxadiazol-5-yl)-4-(trifluoromethyl)pyridine-1-oxide, 2-chloro-3-(3-(3,4-dihydroxy-5-nitrophenyl)-1,2,4-oxadiazol-5-yl)-4,6-dimethylpyridine-1-oxide, 3-(3-(3,4-dihydroxy-5-nitrophenyl)-1,2,4-oxadiazol-5-yl)-2-methyl-6-(trifluoromethyl)pyridine-1-oxide,5-(3-(3,4-dihydroxy-5-nitrophenyl)-1,2,4-oxadiazol-5-yl)-2-(trifluoromethyl)pyridine-1-oxide, 5-(3-(3,4-dihydroxy-5-nitrophenyl)-1 ,2,4-oxadiazol-5-yl)-2-methyl-4-(trifluoromethyl)pyridine-1-oxide, 3-(3-(3,4-dihydroxy-5-nitrophenyl)-1,2,4-oxadiazol-5-yl)-2,6-dimethyl-4-(trifluoromethyl)pyridine-1-oxide, 3,5-dichloro-4-(3-(3,4-dihydroxy-5-nitrophenyl)-1,2,4-oxadiazol-5-yl)pyridine-1-oxide, 3-(3-(3,4-dihydroxy-5-nitrophenyl)-1,2,4-oxadiazol-5-yl)-6-methyl-2-phenyl-4-(trifluoromethyl)pyridine- 1-oxide, 2-bromo-3-(3-(3,4-dihydroxy-5-nitrophenyl)-1,2,4-oxadiazol-5-yl)-4,5,6-trimethylpyridine-1-oxide, 2-chloro-3-(3-(3,4-dihydroxy-5-nitrophenyl)-1,2,4-oxadiazol-5-yl)-4,5,6-trimethylpyridine-1-oxide, 3-(3-(3,4-dihydroxy-5-nitrophenyl)-1,2,4-oxadiazol-5-yl)-2-(trifluoromethyl)pyridine-1-oxide, 2,5-dichloro-3-(3-(3,4-dihydroxy-5-nitrophenyl)-1,2,4-oxadiazol-5-yl)-4,6-dimethylpyridine-1-oxide, 3-(3-(3,4-dihydroxy-5-nitrophenyl)-1,2,4-oxadiazol-5-yl)-5-(trifluoromethyl)pyridine-1-oxide, 3-(3-(3,4-dihydroxy-5-nitrophenyl)-1,2,4-oxadiazol-5-yl)-2-fluoropyridine-1-oxide, 4-(3-(3,4-dihydroxy-5-nitrophenyl)-1,2,4-oxadiazol-5-yl)-2-fluoropyridine-1-oxide, 2-(3-(3,4-dihydroxy-5-nitrophenyl)-1 ,2,4-oxadiazol-5-yl)-6-fluoropyridine-1-oxide, 2-chloro-3-(3-(3,4-dihydroxy-5-nitrophenyl)-1,2,4-oxadiazol-5-yl)-6-methylpyridine 1-oxide, 2-bromo-3-(3-(3,4-dihydroxy-5-nitrophenyl)-1,2,4-oxadiazol-5-yl)-6-methylpyridine-1-oxide, and 2-bromo-5-chloro-3-(3-(3,4-dihydroxy-5-nitrophenyl)-1,2,4-oxadiazol-5-yl)-4,6-dimethylpyridine-1-oxide and their pharmacologically acceptable salts, esters, carbamates or phosphates.

Details of the preparation of compounds of general formula (I) can be found in WO2007/013830A1.

The compounds of general formula (I) may also be present in the form of pharmacologically acceptable salts, esters, carbamates or phosphates thereof. Suitable pharmaceutically acceptable counter ions are known to the art.

It is also possible to use prodrugs of compounds of the general formula (I) in order to alter the therapeutic profile of the active compound.

The compound of formula (I) is administered as a pharmaceutical composition. For the preparation of pharmaceutical compositions of compounds of general formula I, inert pharmaceutically acceptable carriers are admixed with the active compounds. The pharmaceutically acceptable carriers may be solid or liquid. Solid form preparations include powders, tablets, dispersible granules and capsules. A solid carrier can be one or more substances which may also act as diluent, flavouring agent, solubiliser, lubricant, suspending agent, binder, glidant, or disintegrant; it may also be an encapsulating material.

Preferably the pharmaceutical composition is in unit dosage form, e.g. a packaged preparation, the package containing discrete quantities of the preparation, for example packaged tablets, capsules and powders in vials or ampoules.

In general, the compound of formula (I) is administered orally.

The compound of formula (I) typically is administered from once a day to about once weekly.

For the avoidance of doubt, whenever the compound of formula (I) is administered with a periodicity lower than once a day (e.g. once weekly), it is understood that it will be administered prior to sleep, before bedtime or at bedtime, before or after the last daily dose of levodopa of the day(s) of the week where compound (I) should be administered and not every day, as levodopa. For example, for a once weekly administration regime, if compound (I) is administered on the first day of week one, it will be administered prior to sleep, before bedtime or at bedtime, before or after the last daily dose of levodopa of that day. The next administration will occur on the first day of week two, prior to sleep, before bedtime or at bedtime, before or after the last daily dose of levodopa of that day and so on. Throughout this period levodopa is administered every day (in several doses).

According to the methods described above, the compound of formula (I) is typically administered from once a day to about once weekly.

Other aspects of the invention are as defined in the claims.

### Examples

### Example 1: Preparation of compound A

### (5-[3-(2,5-Dichloro-4,6-dimethyl-1-oxy-pyridin-3-yl)-[1,2,4]oxadiazol-5-yl]-3-nitrobenzene-1,2-diol)

a) To a stirred solution of 3,4-dibenzyloxy-5-nitrobenzoic acid (0.50 g, 1.319 mmol) in dimethylformamide (5 mL) at room temperature was added 1,1-carbonyldiimidazole (0.24 g, 1.45 mmol) in one portion. After stirring for ninety minutes, 2,5-dichloro-N'-hydroxy-4,6-dimethylnicotinamide (0.40 g, 1.45 mmol) was added in one portion. The resulting mixture was stirred at 135 °C for five hours and then at room temperature overnight. The reaction mixture was poured onto ice-2 N HCl (100 mL) and the resulting precipitate was filtered off, washed with water and dried in air. Recrystallisation from isopropanol gave a pale yellow solid (0.55 g, 72 %).
b) To a stirred solution of the solid obtained above (0.50 g, 0.866 mmol) in dichloromethane (20 mL) was added urea-hydrogen peroxide addition complex (0.41 g, 4.33 mmol) in one portion. The mixture was cooled in an ice-water bath and trifluoroacetic anhydride (0.73 g, 3.46 mmol) was added dropwise. The reaction mixture was allowed to stir at room temperature overnight whereupon insoluble material was filtered off. The filtrate was washed with water and brine, dried over anhydrous magnesium sulphate, filtered and evaporated. The residue was crystallised from isopropanol to give a pale yellow solid (0.35 g, 68 %).
c) To a stirred solution of the solid obtained above (0.30 g, 0.5 mmol) in dichloromethane (10 mL) at -78 °C under argon was added boron tribromide (0.38 g, 1.5 mmol) dropwise. The resulting purple suspension was allowed to stir at room temperature for one hour, then cooled again to -78 °C and carefully quenched by the addition of water. After stirring at room temperature for one hour, the precipitate was filtered off, washed with water and dried at 50 °C under vacuum to afford the desired compound as yellow crystals (0.18 g, 87 %) of m.p. 237-240 °C.

### Example 2: Pharmaceutical formulation

Pharmaceutical formulations are prepared as follows:

**Capsule:**

| | |
|---|---|
| Compound A | 15.0% |
| Lactose monohydrate | 43.0% |
| Microcrystalline cellulose | 30.0% |
| Povidone | 4.0% |
| Croscarmellose sodium | 5.0% |
| Talc | 2.0% |
| Magnesium stearate | 1.0% |

**Capsule:**

| | |
|---|---|
| Compound A | 15.0% |
| Microcrystalline cellulose | 72.5% |
| Ethylcellulose | 5.0% |
| Sodium starch glycolate | 6.0% |
| Colloidal Silicon Dioxide | 0.5% |
| Magnesium stearate | 1.0% |

**Tablet:**

| | |
|---|---|
| Compound A | 20.0% |
| Microcrystalline cellulose | 25.0% |
| Calcium Phosphate, dibasic dihydrate | 40.0% |
| Povidone | 6.0% |
| Croscarmellose sodium | 6.0% |
| Talc | 2.0% |
| Magnesium stearate | 1.0% |

### Example 3 - Dosing regimen

### Example 3a: Effect of food

This study was an open-label, randomized, single administration. 2-pcriod. 2-sequence, crossover study in 12 healthy male subjects aimed to assess the effects of food on the pharmacokinetic (PK) profile of COMPOUND A and its metabolites. A single 50 mg dose of COMPOUND A (2 x 25 mg capsules) was administered under fasted and fed conditions.

Blood samples were collected prior to and 0.5, 1, 1.5, 2, 3, 4, 6, 8, 10, 12, 16 and 24 hours after administration of COMPOUND A.

### Pharmacokinetic results:

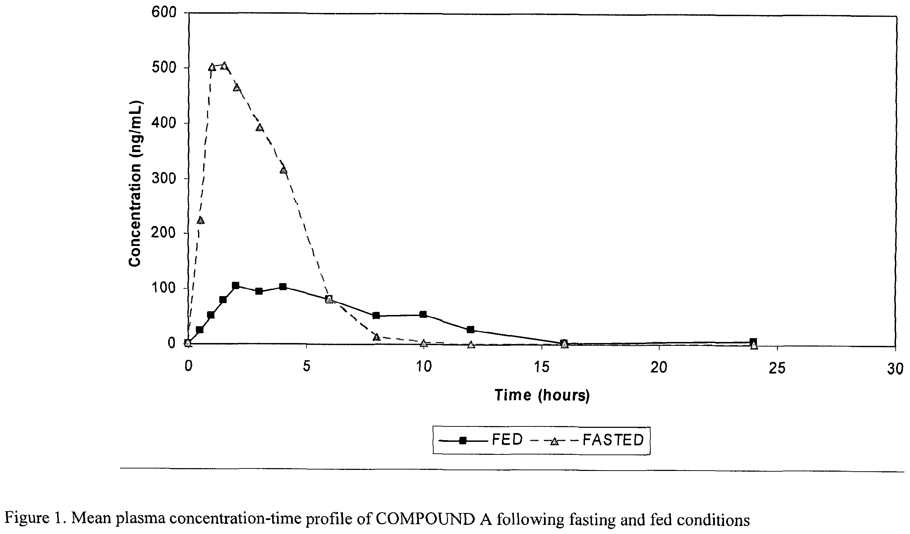

Following an oral single dose of 50 mg (2 x 25 mg capsules), the bioavailability of COMPOUND A was not equivalent under fasting and fed conditions, indicating the presence of a food effect on COMPOUND A pharmacokinetics (Figure 1).

The COMPOUND A pharmacokinetic parameters following fasting and fed conditions are presented in Table 1. Summary of PK main results of COMPOUND A following fasting and fed conditions.

**Table 1. Summary of PK main results of COMPOUND A following fasting and fed conditions.**

| PARAMETER | Fed | | Fasted | | F (treatment) | p* |
|---|---|---|---|---|---|---|
| | MEAN | C.V. (%) | MEAN | C.V. (%) | | |
| Cmax (ng/mL) | 238.2 | 70.7 | 635.0 | 39.5 | 24.84 | <0.01 |
| In (Cₘₐₓ) | 5.2927 | 11.4 | 6.3868 | 5.9 | 41.50 | <0.001 |
| Tₘₐₓ (hours) | 4.00 | 66.3 | 1.50 | 62.9 | 18 | <0.05 |
| AUC_{T} (ng·h/mL) | 879.2 | 32.6 | 1989.5 | 49.5 | 15.95 | <0.01 |
| In (AUC_{T}) | 6.7286 | 5.1 | 7.4900 | 6.4 | 34.22 | <0.001 |
| AUC_{∞} (ng·h/mL) | 1027.2 | 53.1 | 2113.6 | 43.3 | 36.53 | <0.001 |
| In (AUC_{∞}) | 6.8416 | 6.2 | 7.5771 | 5.5 | 52.24 | <0.001 |
| AUC_{T/∞} (%) | 90.06 | 12.1 | 93.49 | 17.0 | 0.75 | N.S. |
| Kₑₗ (hours⁻¹) | 0.4021 | 46.0 | 0.7581 | 38.9 | 15.99 | <0.01 |
| T_{½el} (hours) | 3.35 | 141.2 | 1.19 | 80.5 | 2.19 | N.S. |

| | | | | | | |
|---|---|---|---|---|---|---|
| * N.S.= Not Significant. Significant whenever p-value < 0.05; ^{§} For Tₘₐₓ, the median is presented and the statistical analysis is based on a non-parametric approach. C.V. - Coefficient of Variation | | | | | | |

The ingestion of food decreased and delayed the absorption of COMPOUND A; the administration of COMPOUND A immediately following consumption of a high-fat, high-calorie meal significantly decreased the rate and extent of absorption compared to drug administration under fasting conditions. Cₘₐₓ and AUC parameters were significantly lower in the fed state compared to the fasted state (fed:fasted ratios of 31.73% for Cₘₐₓ, 47.11% for AUC_{T} and 49.43% for AUC_{∞}). The Tₘₐₓ was significantly increased by the presence of food (4.00 hours in the fed state versus 1.50 hours in the fasted state).

### Conclusions:

The results show that the administration of COMPOUND A following food intake results in a reduction in its bioavailability. The presence of food decreased the rate and extent of absorption of COMPOUND A, with delayed peak plasma levels as compared to drug administration under fasting conditions.

### Example 3b: Administration of levodopa and Compound A concomitantly and separated by 1 hour

This study was a single-centre, open-label, randomized, gender-balanced, crossover study with four consecutive single-administration treatment periods to assess the PK-PD interaction when standard release 25/100 mg carbidopa/levodopa is administered concomitantly with a 50 mg COMPOUND A dose or 1 hour thereafter. Eighteen (18) subjects completed 2 treatment periods, 17 subjects completed 3 treatment periods and 16 subjects completed all 4 treatment periods. A total of 18 male [10 (55.6%)] and female [8 (44.4%)] subjects were enrolled in this study.

Treatment consisted of four single-administration periods. Single-administration of 50 mg COMPOUND A was constituted by 2 capsules of 25 mg. Single-administration of immediate/standard release 25 mg carbidopa / 100 mg levodopa was constituted of 1 tablet of Sinemet® 100/25. In accordance to the treatment sequence defined by the randomization, subjects were administered COMPOUND A and Sinemet® 100/25 concomitantly in one period, Sinemet® 100/25 1 h after the COMPOUND A administration in another period, COMPOUND A alone in another period, and Sinemet® 100/25 alone in the remaining period. The washout period between administrations was at least 3 weeks. In one treatment period, COMPOUND A and Sinemet® 100/25 were to be administered concomitantly; in another treatment period, Sinemet® 100/25 was to be administered 1 h after the COMPOUND A administration; in another treatment period, COMPOUND A was to be administered alone; in the remaining treatment period, Sinemet® 100/25 was to be administered alone.

Mean levodopa Cₘₐₓ values were attained between 0.5 to 1.0 hours post-administration. Thereafter, plasma levodopa concentrations declined with a mean elimination half-life (t1/2) ranging from 1.94 (Sinemet® 100/25 alone) to 2.51 (Sinemet® 100/25 plus 50 mg COMPOUND A concomitantly) hours.

Following administration with COMPOUND A, the levodopa Cₘₐₓ increased and the increase was higher when COMPOUND A was administered concomitantly, suggesting that a certain degree of interaction between COMPOUND A and Sinemet® 100/25 may have occurred during the absorption phase, leading to an increase in the rate of levodopa uptake and a less smooth increase in levodopa levels.

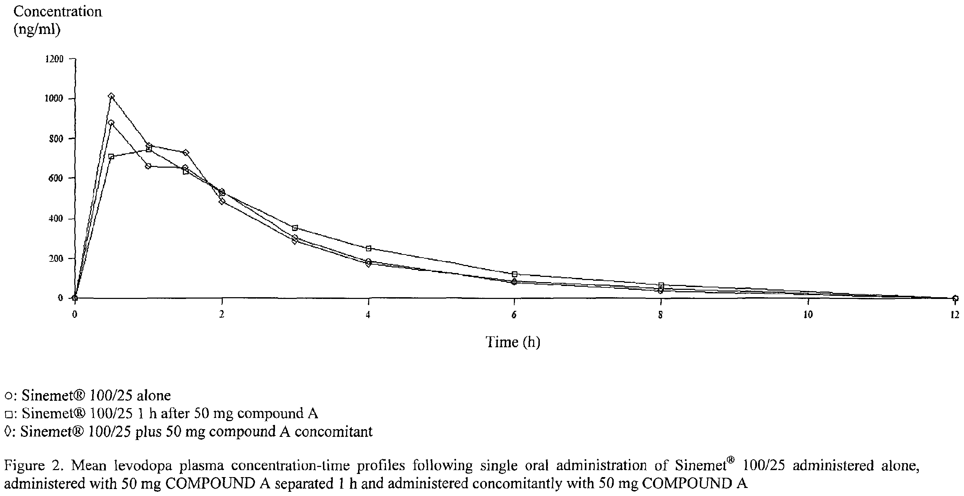

Mean levodopa plasma concentration-time profiles following single oral administration of Sinemet® 100/25 administered alone, administered with 50 mg COMPOUND A separated 1h and administered concomitantly with 50 mg COMPOUND A are illustrated in Figure 2 (n=16 for concomitant administration, n=17 for Sinemet® alone, n=18 for administration separated 1 h):

The Point Estimates and 90% Confidence Interval of the mean pharmacokinetic parameters of levodopa following 50 mg COMPOUND A concomitant administration (*Test L1*) and an administration separated 1 h (*Test L2*) with Sinemet® 100/25 are displayed in Table 2 (Sinemet® 100/25 alone was taken as *Reference*):

**Table 2 Point estimates and 90%CI of the mean pharmacokinetic parameters of levodopa following 50 mg COMPOUND A concomitant administration (Test L1) and an administration separated 1 h (Test L2) with Sinemet® 100/25**

| Comparison | Cₘₐₓ PE (90%CI) | AUC₀₋ₜ PE (90%CI) | AUC_{0-∞} PE (90%Cl) |
|---|---|---|---|
| *Test L1*/*Reference L* | 112.10 (96.94; 129.64) | 104.23 (96.88; 112.14) | 103.13 (94.02; 113.12) |
| *Test L2*/*Reference L* | 102.96 (89.36; 118.62) | 114.56 (106.65; 123.05) | 109.85 (100.22; 120.41) |
| *Test L2lTest L1* | 91.84 (79.51: 106.09) | 109.91 (102.17; 118.24) | 108.51 (101.24; 116.31) |

| | | | |
|---|---|---|---|
| PE = Point estimate; CI = Confidence interval | | | |

A greater increase in the extent of exposure to levodopa (as assessed by AUC) occurred when Sinemet® 100/25 was administered 1 h after 50 mg COMPOUND A.

The increase in levodopa AUC₀₋ₜ ranged from 4.23% when Sinemet® 100/25 was administered concomitantly with 50 mg COMPOUND A (ratios = 104.23 [96.88; 112.14]; means and 90%CIs) to 14.56% when Sinemet® 100/25 was administered 1 h after 50 mg COMPOUND A (ratios = 114.56 [106.65; 123.05]). The increase in levodopa AUC_{0-∞} ranged from 3.13% when Sinemet® 100/25 was administered concomitantly with 50 mg COMPOUND A (ratios = 103.13 [94.02; 113.12]) to 9.85% when Sinemet® 100/25 was administered 1 h after 50 mg COMPOUND A (ratios = 109.85 [100.22; 120.41]).

The increase in levodopa Cₘₐₓ ranged from 2.96% when Sinemet® 100/25 was administered 1 h after 50 mg COMPOUND A (ratios = 102.96 [89.36; 118.62]) to 12.10% when Sinemet® 100/25 was administered concomitantly with 50 mg COMPOUND A (ratios = 112.10 [96.94; 129.64]), showing a preferred steadier increase in levodopa levels with administration of Sinemet® 100/25 was administered 1 h after COMPOUND A.

Cₘₐₓ of 3-OMD was lower when Sinemet® 100/25 was administered 1 h after 50 mg COMPOUND A than when Sinemet® 100/25 was administered concomitantly with 50 mg COMPOUND A. A significant decrease in both rate (as assessed by Cₘₐₓ) and extent (as assessed by AUC) of systemic exposure to 3-OMD occurred when Sinemet® 100/25 was administered concomitantly and also 1 h after 50 mg COMPOUND A.

Cₘₐₓ and AUC of carbidopa were similar when Sinemet® 100/25 was administered alone and when administered with 50 mg COMPOUND A separated 1 h. The increase in carbidopa Cₘₐₓ ranged from 5.33% when Sinemet® 100/25 was administered 1 h after 50 mg COMPOUND A to 5.86% when Sinemet® 100/25 was administered concomitantly with 50 mg COMPOUND A. The increase in carbidopa AUC_{0-∞} ranged from 5.42% when Sinemet® 100/25 was administered 1 h after 50 mg COMPOUND A to 9.20% when Sinemet® 100/25 was administered concomitantly with 50 mg COMPOUND A.

Mean COMPOUND A Cₘₐₓ values were attained between 2.5 to 4.0 hours post-administration. Thereafter, plasma COMPOUND A concentrations declined with a mean elimination half-life (tl/2) ranging from 1.14 (COMPOUND A alone) to 1.28 (COMPOUND A with Sinemet® 100/25) hours. Cₘₐₓ of COMPOUND A was lower when COMPOUND A was administered 1 h before Sinemet® 100/25 than when was administered concomitantly with Sinemet® 100/25 and even lower in relation to when COMPOUND A was administered alone. Following administration with Sinemet® 100/25, the COMPOUND A Cₘₐₓ decreased and tₘₐₓ increased, suggesting that a certain degree of interaction between COMPOUND A and Sinemet® 100/25 may have occurred during the absorption phase, leading to a delay in the rate of COMPOUND A uptake. A statistical difference was found for tₘₐₓ between 50 mg COMPOUND A administered concomitantly with Sinemet® 100/25 and COMPOUND A administered alone (p-0.0020). The increase in COMPOUND A AUC_{0-∞} ranged from 4.18% when Sinemet® 100/25 was administered concomitantly with 50 mg COMPOUND A to 4.74% when Sinemet® 100/25 was administered 1h after 50 mg COMPOUND A. There was a decrease in COMPOUND A Cₘₐₓ of 9.21% when Sinemet® 100/25 was administered 1 h after 50 mg COMPOUND A.

### Pharmacodynamic results:

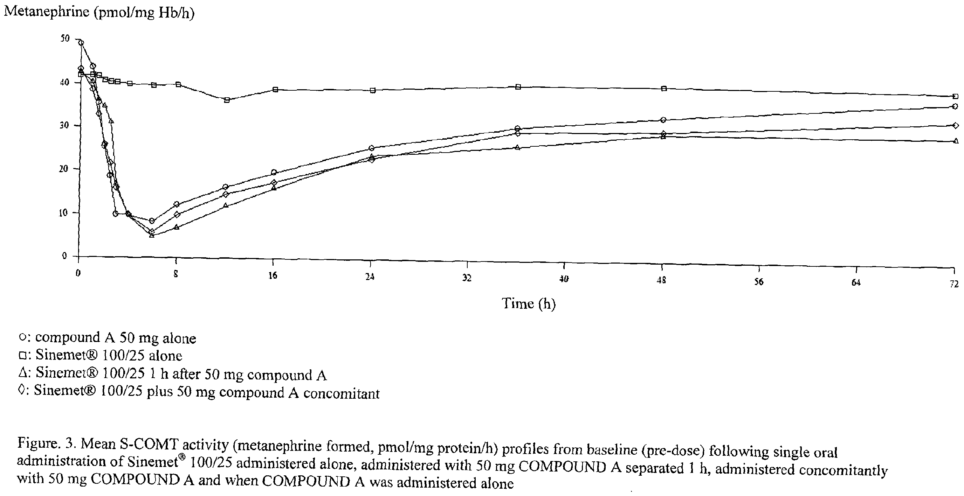

Mean S-COMT activity (metancphrine formed, pmol/mg protein/h) profiles from baseline (pre-administration) following single oral administration of Sinemet® 100/25 administered atone, administered with 50 mg COMPOUND A separated by 1 h. administered concomitantly with 50 mg COMPOUND A and when COMPOUND A was administered alone were as follows (Figure 3) (n=16 for concomitant administration, n=17 for Sinemet® alone, n=18 for COMPOUND A alone and for administration separated by 1 h):
All COMPOUND A treatments significantly inhibited both peak and extent of S-COMT activity in relation to Sinemet® 100/25 alone administration. Following administration of COMPOUND A, maximum S-COMT inhibition (Eₘₐₓ) occurred between 3.42 h (COMPOUND A alone) and 4.58 h (Sinemet® 100/25 administered 1 h after 50 mg COMPOUND A) post-administration (tEₘₐₓ), and ranged from 88.7% to 91.1%, respectively.

### Conclusions:

The results were highly consistent across the multiple analyses performed. All COMPOUND A treatments significantly inhibited both peak and extent of S-COMT activity in relation to Sinemet® 100/25 alone administration. The concomitant administration of COMPOUND A with Sinemet® 100/25 showed an effect on both levodopa and COMPOUND A absorption. In the latter, as well as the slight Cₘₐₓ decrease, a significant increase in COMPOUND A tₘₐₓ was observed. With respect to levodopa, delaying by 1 hour the administration with COMPOUND A, a Cₘₐₓ decrease (when compared to the increase observed with the concomitant administration) was seen, suggesting that the possible interaction between COMPOUND A and Sinemet® 100/25 during the absorption phase was reduced by separating both administrations. Furthermore, delaying by 1 hour the administration of levodopa with COMPOUND A, an increase in levodopa systemic exposure (as assessed by AUC) was observed. This could be attributed to the absorption rate of COMPOUND A and consequently to the inhibition of COMT. Indeed, and despite the significant increase in the t_{Emax} for both treatments with COMPOUND A and Sinemet® 100/25 in relation to the administration of COMPOUND A alone, the 1 hour separated administration induced a more sustained absorption rate of COMPOUND A in contrast to the abrupt and delayed absorption observed with the concomitant administration. This may have resulted in an early inhibition of COMT and consequent increase in levodopa systemic exposure.

### Example 3c: Effect of compound A on patient's levodopa exposure after administration of L-DOPA and COMPOUND A concomitantly followed by further administration of L-DOPA 24 h later.

This study was a three-center, double-blind, randomised, placebo-controlled, cross-over study to investigate the tolerability and effect a single administration of three dosages of *COMPOUND A* (25, 50 and 100mg) on the levodopa pharmacokinetics, motor response, and erythrocyte soluble catechol-O-methyltransferase activity in 10 Parkinson's Disease patients concomitantly treated with levodopa/dopa-decarboxylase inhibitor.

Subjects were eligible if they presented: a diagnosis of PD according to the UK PDS Brain Bank diagnostic criteria; predictable signs of end-of-dose deterioration despite "optimal" levodopa/AADCi therapy; being treated with a stable regimen of 3 to 8 doses of standard release levodopa/ AADCi 100/25 mg per day within at least 1 week prior to randomisation; modified Hoehn and Yahr stage of less than 5 in the off-state; and/or mean duration of OFF stage ≥1.5 h during waking hours. Concomitant anti-Parkinsonian medication (other than apomorphine, entacapone or tolcapone) was allowed in stable doses for at least 4 weeks prior to randomisation.

Manipulating the dose and frequency of levodopa administration is the common therapeutic approach to the onset of motor complications. This is usually described as optimization of levodopa therapy. "Optimal" levodopa/AADCi therapy is the levodopa/AADCi dosage and administration regime, which produces the best motor response in a patient, i.e. absence or reduction to a minimum of end-of-dose deterioration (wearing-off) and/or motor complications.

The study consisted of four consecutive treatment periods, corresponding to the 4 different treatment options (Compound A 25 mg, 50 mg, 100 mg or placebo). In each of the four treatment periods, subjects were to be admitted to the study site 2 days prior to receiving the administration of Compound A/Placebo (Day 1) and were to remain hospitalized ("in-patient") until 48 h after receiving the administration of Compound A/Placebo. The washout period between administrations was to be at least 10 days. A follow-up visit was to occur approximately 2 weeks after the last treatment administration or early discontinuation. During each period, the *COMPOUND* A/Placebo capsules were to be co-administered with the morning dose of levodopa/carbidopa 100/25 mg (1 tablet of Sinemet® 25/100) or levodopa/benserazide 100/25 mg (1 tablet of Madopar®/Restex® 125) on Day 3.

A total of 10 subjects were enrolled in this study: 10 subjects completed 3 treatment periods and 9 subjects completed all 4 treatment periods. The mean (±SD) age, height and weight were 58.40±10.24 (range: 42-70) years, 1.69±0.14 (1.52-1.95) m, 71.5±15.06 (50-100) kg, respectively.

Results from this study can be found in Table 3 and Table 4.

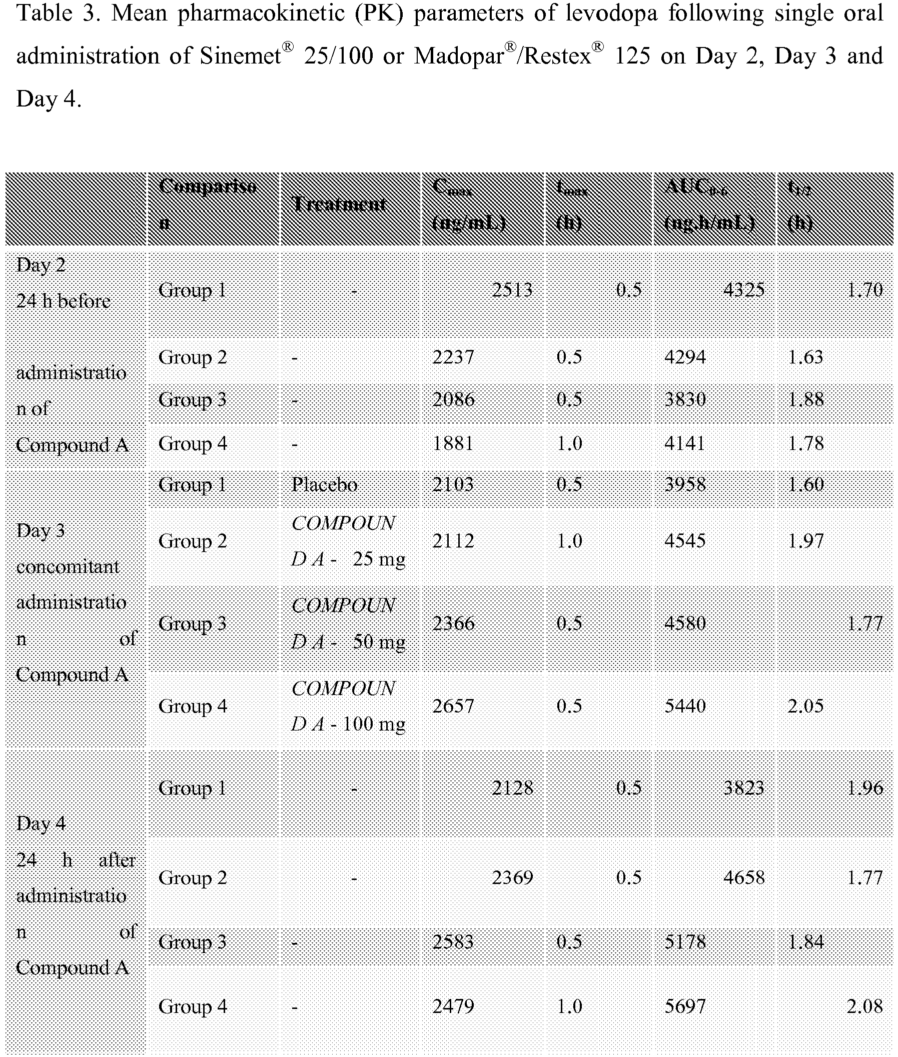

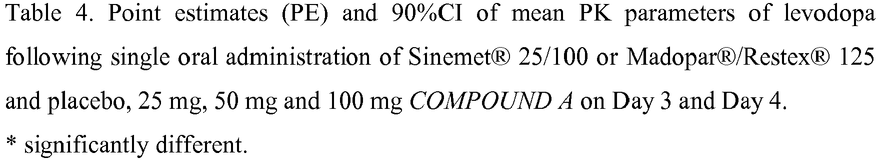

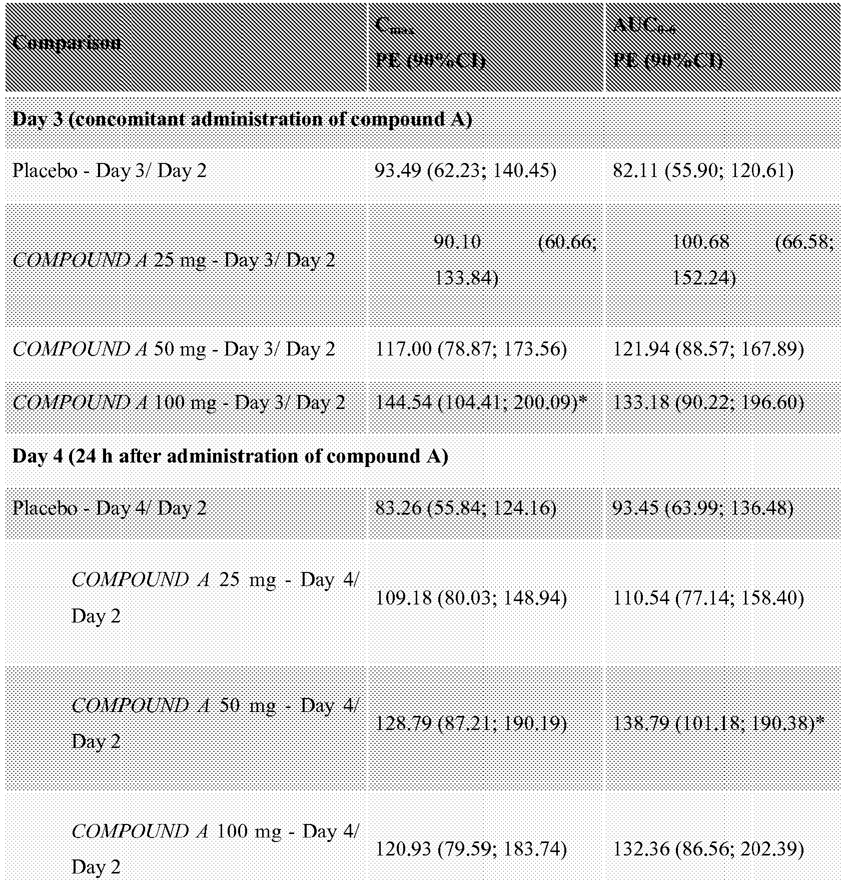

### Example 3d: Clinical trial in patients with Parkinson's disease: dosage prior to sleep

In this study, the Compound A is tested as the research therapy and entacapone and placebo as the reference therapies. Compound A is available in capsules of 5 mg, 25 mg and 50 mg. Entacapone tablets of 200 mg are used. To ensure blinding during the Double Blind (DB) period, the Compound A capsules and entacapone tablets are identically over-encapsulated. The placebo capsules are prepared by filling identical capsules with filler (also used as back-filling). All placebo capsules contain, on average, 1 mg riboflavin to mimic the urinary discoloration seen as a harmless side effect of entacapone

### Dosage Schedule

During the DB period, subjects take 1 capsule of treatment concomitantly with each daytime levodopa/AADCi administration (3 to 8 daily administrations). An additional treatment ('before bedtime' or 'prior to sleep' administration) is administered at least 1 hour after the last administration of the day of levodopa/AADCi.

For the daytime administrations (i.e. taken concomitantly with each levodopa/AADCi administration), the treatment administration is as follows:
- Compound A Groups: placebo.
- Entacapone Group: 200 mg entacapone.
- Placebo Group: placebo.

For the bedtime dose (at least 1 hour after the last daily administration of levodopa/AADCi), the treatment administration is as follows:
- Compound A Groups: 5, 25 or 50 mg.
- Entacapone Group: placebo.
- Placebo Group: placebo.

### Example 3e: Clinical trial in patients with Parkinson's disease: dosage prior to sleep and 1 hour after food intake

In a double blind, placebo controlled study, patients with Parkinson's disease maintained on levodopa/AADCi are treated as follows. Patients take either the placebo or compound A (25 mg or 50 mg) in the evening at least one hour after the last dose of the day of levodopa/AADCi therapy (the bedtime dose (administration)).

Subjects are required to fast for 1 hour before and for at least 1 hour after intake of the treatment.

Patients who take compound A are expected to show improved effects relative to those taking the placebo.

## Claims

1. The compound 5-[3-(2,5-dichloro-4,6-dimethyl-1-oxy-pyridin-3-yl)-[1,2,4]oxadiazol-5-yl]-3-nitrobenzene-1,2-diol or its pharmacologically acceptable salts, esters, carbamates and phosphates, for use in combination with the catecholamine drug levodopa, for the prophylaxis or treatment of Parkinson's disease, wherein the compound is administered orally in a single daily dose at least one hour before or after the last daily dose of the catecholamine drug.

2. The compound for use according to claim 1, wherein the effective daily dose of the compound is 25 to 300 mg/day.

3. The compound for use according to claim 1, wherein the specific daily dose of the compound is 1 mg, 3 mg, 5 mg, 10 mg, 15 mg, 20 mg, 25 mg, 30 mg, 50 mg, 100 mg, 200 mg, 400 mg, 800 mg or 1200 mg.

4. The compound for use according to claim 1, wherein the specific daily dose of the compound is 50 mg.

5. The compound for use according to one of claims 1 to 4, wherein the compound is administered from 0.25 to 12 hours, preferably from 0.5 to 6 hours, more preferably from 0.75 to 4 hours, after an intake of food.

6. The compound for use according to any one of claims 1 to 5, wherein the compound is administered from 0.25 to 2 hours, preferably from 0.5 to 1.5 hours, before an intake of food.

7. The compound for use according to any one of claims 1 to 4, wherein the compound is administered without food and/or at least one hour after the most recent intake of food and at least one hour before the next intake of food.

8. The compound for use according to any of claims 1 to 7, wherein the compound is administered less than 90 minutes before going to bed, preferably less than one hour before going to bed.

9. The compound for use according to any of claims 1 to 8, wherein the compound is administered at bedtime.

10. The compound for use according to any one of claims 1 to 9, wherein the subsequent dose of the catecholamine is at least two, preferably at least six hours after the administration of the compound.

11. The compound for use according to any one of claims 1 to 10, wherein the catecholamine drug is administered sequentially or concomitantly with an AADCi, such as carbidopa or benserazide.

12. The compound for use according to any one of claims 1 to 11, wherein the Parkinson's disease is Parkinson's disease with motor complications.

13. The compound 5-[3-(2,5-dichloro-4,6-dimethyl-1-oxy-pyridin-3-yl)-[1,2,4]oxadiazol-5-yl]-3-nitrobenzene-1,2-diol or its pharmacologically acceptable salts, esters, carbamates and phosphates, for use in combination with the catecholamine drug levodopa, for the prophylaxis or treatment of Parkinson's disease, wherein the compound is administered orally once daily at least one hour before or after the last daily dose of the catecholamine drug, and less than 90 minutes before going to bed or at bedtime, and at least one hour after the most recent intake of food and at least one hour before the next intake of food.

## Patentansprüche

1. Verbindung 5-[3-(2,5-Dichlor-4,6-dimethyl-1-oxy-pyridin-3-yl)-[1,2,4]oxadiazol-5-yl]-3-nitrobenzol-1,2-diol oder ihre pharmakologisch annehmbaren Salze, Ester, Carbamate und Phosphate zur Verwendung in Kombination mit dem Katecholamin-Arzneimittel Levodopa zur Prophylaxe oder Behandlung von Parkinson-Krankheit, wobei die Verbindung oral in einer einzigen Tagesdosis mindestens eine Stunde vor oder nach der letzten Tagesdosis des Katecholamin-Arzneimittels verabreicht wird.

2. Verbindung zur Verwendung nach Anspruch 1, wobei die wirksame Tagesdosis der Verbindung 25 bis 300 mg/Tag ist.

3. Verbindung zur Verwendung nach Anspruch 1, wobei die spezifische Tagesdosis der Verbindung 1 mg, 3 mg, 5 mg, 10 mg, 15 mg, 20 mg, 25 mg, 30 mg, 50 mg, 100 mg, 200 mg, 400 mg, 800 mg oder 1200 mg ist.

4. Verbindung zur Verwendung nach Anspruch 1, wobei die spezifische Tagesdosis der Verbindung 50 mg ist.

5. Verbindung zur Verwendung nach einem der Ansprüche 1 bis 4, wobei die Verbindung 0,25 bis 12 Stunden, vorzugsweise 0,5 bis 6 Stunden, bevorzugter 0,75 bis 4 Stunden, nach einer Nahrungsaufnahme verabreicht wird.

6. Verbindung zur Verwendung nach einem der Ansprüche 1 bis 5, wobei die Verbindung 0,25 bis 2 Stunden, vorzugsweise 0,5 bis 1,5 Stunden, vor einer Nahrungsaufnahme verabreicht wird.

7. Verbindung zur Verwendung nach einem der Ansprüche 1 bis 4, wobei die Verbindung ohne Nahrung und/oder mindestens eine Stunde nach der jüngsten Nahrungsaufnahme und mindestens eine Stunde vor der nächsten Nahrungsaufnahme verabreicht wird.

8. Verbindung zur Verwendung nach einem der Ansprüche 1 bis 7, wobei die Verbindung weniger als 90 Minuten vor dem Schlafengehen, vorzugsweise weniger als eine Stunde vor dem Schlafengehen, verabreicht wird.

9. Verbindung zur Verwendung nach einem der Ansprüche 1 bis 8, wobei die Verbindung zur Schlafenszeit verabreicht wird.

10. Verbindung zur Verwendung nach einem der Ansprüche 1 bis 9, wobei die nachfolgende Dosis des Katecholamins mindestens zwei, vorzugsweise mindestens sechs Stunden nach der Verabreichung der Verbindung ist.

11. Verbindung zur Verwendung nach einem der Ansprüche 1 bis 10, wobei das Katecholamin-Arzneimittel nacheinander oder gleichzeitig mit einem AADCi, wie beispielsweise Carbidopa oder Benserazid, verabreicht wird.

12. Verbindung zur Verwendung nach einem der Ansprüche 1 bis 11, wobei die Parkinson-Krankheit eine Parkinson-Krankheit mit motorischen Komplikationen ist.

13. Verbindung 5-[3-(2,5-Dichlor-4,6-dimethyl-1-oxy-pyridin-3-yl)-[1,2,4]oxadiazol-5-yl]-3-nitrobenzol-1,2-diol oder ihre pharmakologisch annehmbaren Salze, Ester, Carbamate und Phosphate zur Verwendung in Kombination mit dem Katecholamin-Arzneimittel Levodopa zur Prophylaxe oder Behandlung von Parkinson-Krankheit, wobei die Verbindung oral einmal täglich mindestens eine Stunde vor oder nach der letzten Tagesdosis des Katecholamin-Arzneimittels und weniger als 90 Minuten vor dem Schlafengehen oder zur Schlafenszeit und mindestens eine Stunde nach der jüngsten Nahrungsaufnahme und mindestens eine Stunde vor der nächsten Nahrungsaufnahme verabreicht wird.

## Revendications

1. Composé 5-[3-(2,5-dichloro-4,6-diméthyl-1-oxy-pyridin-3-yl)-[1,2,4]oxadiazol-5-yl]-3-nitrobenzène-1,2-d iol ou ses sels, esters, carbamates et phosphates pharmacologiquement acceptables, pour l'utilisation en association avec le médicament catécholamine lévodopa, pour la prophylaxie ou le traitement de la maladie de Parkinson, le composé étant administré par voie orale en une dose quotidienne unique au moins une heure avant ou après la dernière dose quotidienne du médicament catécholamine.

2. Composé pour l'utilisation selon la revendication 1, la quantité quotidienne efficace du composé étant 25 à 300 mg/jour.

3. Composé pour l'utilisation selon la revendication 1, la dose quotidienne spécifique du composé étant 1 mg, 3 mg, 5 mg, 10 mg, 15 mg, 20 mg, 25 mg, 30 mg, 50 mg, 100 mg, 200 mg, 400 mg, 800 mg ou 1 200 mg.

4. Composé pour l'utilisation selon la revendication 1, la dose quotidienne spécifique du composé étant 50 mg.

5. Composé pour l'utilisation selon l'une des revendications 1 à 4, le composé étant administré 0,25 à 12 heures, de préférence 0,5 à 6 heures, plus préférablement 0,75 à 4 heures, après une prise de nourriture.

6. Composé pour l'utilisation selon une quelconque des revendications 1 à 5, le composé étant administré 0,25 à 2 heures, de préférence 0,5 à 1,5 heure, avant une prise de nourriture.

7. Composé pour l'utilisation selon une quelconque des revendications 1 à 4, le composé étant administré sans nourriture et/ou au moins une heure après prise de nourriture la plus récente et au moins une heure avant la prochaine prise de nourriture.

8. Composé pour l'utilisation selon une quelconque des revendications 1 à 7, le composé étant administré moins de 90 minutes avant le coucher, de préférence moins d'une heure avant le coucher.

9. Composé pour l'utilisation selon une quelconque des revendications 1 à 8, le composé étant administré au moment du coucher.

10. Composé pour l'utilisation selon une quelconque des revendications 1 à 9, la dose subséquente de la catécholamine étant prise au moins deux, de préférence au moins six heures après l'administration du composé.

11. Composé pour l'utilisation selon une quelconque des revendications 1 à 10, le médicament catécholamine étant administré séquentiellement ou concomittament avec un AADCi, tel que la carbidopa ou le bensérazide.

12. Composé pour l'utilisation selon une quelconque des revendications 1 à 11, la maladie de Parkinson étant une maladie de Parkinson avec des complications motrices.

13. Composé 5-[3-(2,5-dichloro-4,6-diméthyl-1-oxy-pyridin-3-yl)-[1,2,4]oxadiazol-5-yl]-3-nitrobenzène-1,2-d iol ou ses sels, esters, carbamates et phosphates pharmacologiquement acceptables, pour l'utilisation en association avec le médicament catécholamine lévodopa, pour la prophylaxie ou le traitement de la maladie de Parkinson, le composé étant administré par voie orale une fois par jour au moins une heure avant ou après la dernière dose quotidienne du médicament catécholamine, et moins de 90 minutes avant le coucher ou à l'heure du coucher, et au moins une heure après la prise de nourriture la plus récente et au moins une heure avant la prochaine prise de nourriture.
